Europäisches Patentamt

European Patent Office

Office européen des brevets

(11) Publication number: 0 289 314 B1

# EUROPEAN PATENT SPECIFICATION

(45) Date of publication of patent specification: **12.10.94** (51) Int. Cl.⁵: **A61K 37/36**, A61K 33/16

(21) Application number: **88303855.6**

(22) Date of filing: **28.04.88**

(54) **Use of IGF-II in the treatment of bone disorders.**

(30) Priority: **06.05.87 GB 8710676**
**28.04.87 US 43628**

(43) Date of publication of application:
**02.11.88 Bulletin 88/44**

(45) Publication of the grant of the patent:
**12.10.94 Bulletin 94/41**

(84) Designated Contracting States:
**AT BE CH DE ES FR GB GR IT LI LU NL SE**

(56) References cited:
**EP-A- 0 128 733          EP-A- 0 224 885**
**EP-A- 0 280 460          WO-A-85/00831**
**DE-A- 3 343 253          US-A- 3 287 219**

**R.K. Schenk et al., Perspectives on
Biomaterials, ed. O.C.C. Lin and E.Y.S. Chao,
1986, Elsevier Publ., pages 80-81**

**CALCIFIED TISSUE INTERNATIONAL, vol. 35,
1983, pages 578-585, Springer Verlag; Ch.
SCHMID et al.: "Insulin-like growth factors
stimulate synthesis of nucleic acids and gly-
cogen in cultured calvaria cells"**

(73) Proprietor: **BOEHRINGER MANNHEIM GMBH**

**D-68298 Mannheim (DE)**

(72) Inventor: **Baylink David J.**
**1120 Benton Street**
**Loma Linda, California 93257 (US)**

EP 0 289 314 B1

ACTA ENDOCRINOLOGICA, vol. 106, 1985, pages 167-174; E. SCHOENLE et al.: "Comparison of in vivo effects of insulin-like growth factors I and II and of growth hormone in hypophysectomized rats"

J. CLIN. INVEST., vol. 77, June 1986, pages 1903-1908, The Am. Soc. for Clin. Invest. Inc. U. VETTER et al.: "Human fetal and adult chondrocytes. Effect of insulin-like growth factors I and II, insulin, and growth hormone on clonal growth"

J. OF CLIN. ENDOCRINOLOGY AND METABOLISM, vol. 59, no. 4, pages 701-704, The Endocrine Soc. US; A.E. BENNETT et al.: "Insulin-like growth facts I and II: aging and bone density in women"

METABOL. CLIN. EXP., vol. 37, no. 10, October 1988, pages 988-995; J.R. FARLEY et al.: "Evidence that fluoride-stimulated3(H)-thymidine incorporation in embryonic chick calvarial cell cultures is dependent on the presence of a bone cell mitogen, sensitive to changes in the phosphate concentration, and modulated by systemic skeletal effectors"

CLINICS IN ENDOCRINOLOGY AND METABOLISM, vol. 13, no. 1, March 1984, pages 3-30 J. ZAPF et al.: "Biological and immunological properties of insulin-like growth factors (IGF) I and II"

CLINICAL RESEARCH, vol. 32, no. 1, 1984, page 48A, C.A. HOWARD et al.: "Somatomedins directly stimulate osteoblast proliferation in vitro"

**Description**

This invention relates to the use of insulin-related growth factor-II (IGF-II) in pharmacological circumstances.

IGF-II belongs to the family of growth factors that also comprises insulin, relaxin, insulin-like growth factor-I (IGF-I) and possibly the beta sub-unit of the 7s nerve growth factor - see Blundell and Humbel, Nature **287** 781 (1980). IGF-II differs from the other members of its family by molecular weight amino acid sequence and the length of its connecting peptide. The primary structure of human IGF-II was first elucidated by Rinderknecht and Humbel, FEBS Letters **89** 283 (1978). They give the amino acid sequence as:


AYRPSETLCGGELVDTLQFVCGDRGFYFSRPASRVSRRSRGIVEECCFRS

CDLALLETYCATPAKSE


In the above structure, and throughout this specification, the single letter amino acid code can be correlated with corresponding three-letter notation as follows:


D=Asp E=Glu F=Phe K=Lys N=Asn
Q=Gln R=Arg W=Trp Y=Tyr M=Met
C=Cys L=Leu I=Ile H=His T=Thr
S=Ser P=Pro G=Gly V=Val A=Ala


The physiological role of IGF-II has been the subject of much speculation and little evidence. However, Brown et al, J. Receptor Res. **5** 297 (1985) have shown that physiological concentrations of IGF-II stimulate thymidine incorporation by primary cultures of activated human T lymphocytes. IGF-II has been shown to enhance erythroid colony formation by human marrow cells (Dainniak and Kreczuko J. Clin. Invest. **76** 1237 (1985)) and may be involved in the beta transforming growth factor-induced transformation of normal rat kidney (NRK) cells in soft agar (Massague et al, J. Biol. Chem. **260** 455 (1985). Serum levels of IGF-II determined by radio immunoassays or radio receptor assays were not significantly different in acromegalics or growth hormone deficient subjects but are significantly elevated in the third trimester of pregnancy to return to lower levels after delivery (Wilson et al, J. Clin. Endo. Meta. **55** 858 1982). There is a decline in serum IGF-II levels with age in normal women (644 ± 136 ng/ml in the eighth decade v 723 ± 217 ng/ml in the third decade, N = 57).

In spite of these observations, Zumstein et al (in PNAS **82** 3169 (1985)) were moved to say that though IGF-I appeared to be a growth factor mediating the growth effects of and being under control of growth hormone, "the function of IGF-II is less clear".

Different animals may have different IGF-II molecules. In addition, Zumstein et al (Op. cit.) have identified and sequenced a variant IGF-II.

EP-A-0135094 discloses the amino acid sequence of IGF-I and IGF-II and nucleotide sequences of genes encoding them. Various hypotheses for their utility are put forward, but nothing concrete by way of clinically ueful activity is proposed, at least for IGF-II, other than the treatment of pituitary dwarfism.

WO-A-8600619 discloses prepro IGF-I and prepro IGF-II, but again gives no clinical utility for the end peptides.

EP-A-0193112 discloses cDNA encoding IGF-II. Again, no specific clinical utility is disclosed or forecast for IGF-II.

EP-A-0128733 discloses the production of "various forms" of human IGF and EGF (Epidermal Growth Factor) by recombinant DNA technology. There is no specific disclosure of clinical utility beyond saying that human IGF can be used as a human growth factor.

E. Schoenle et al. (Acta Endocrinologica 108 (1985) 167 - 174) compare the in vivo effects of IGF I and IGF II in hypophysectomized rats on influencing cartilage growth. Only IGF I exhibits a distinct influence on cartilage growth, whereas IGF II hardly shows any effect.

DE-A 33 43 253 describes the use of fluoride in the treatment of osteoporosis.

It has now been discovered that IGF-II can be of use in the treatment of bone disorders.

For several years it has been suspected that differentiation of bone forming tissues and the growth of differentiated bone is the result of the action of local tissue factors and that the coupling of bone formation to resorption provides a local mechanism for site-specific maintenance of endosteal bone volume. Illustrative publications on this subject include:

Farley et al, Program and Abstracts of 61st Annual Meeting of the Endocrine Society (1979);

Howard & Baylink, Clinical Research **28** 50A (1980);

Howard et al, Calcif. Tissue Int. **31** (Suppl.) 53 (1980);

Baylink & Liu, J. Periodontol. **50** 43-49 (1979);

Ivey & Baylink (Editorial) Metab. Bone Dis. Relat. Res. **3** 3-7 (1981);

Harris & Haeny, New Engl. J. Med. **280** 253-259 (1969);

Howard et al, Metab. Bone Dis. Relat. Res. **2** 131-135 (1980);

Howard et al, PNAS USA **78** 3204-3208 (1981);

Drivdhal et al, Proc. Soc. Exp. Biol. Med. **168** 143-150 (1981) and

Drivdhal et al, Biochem. Biophys. Acta **714** 26-33 (1982).

The possibility of there being a localised factor which controls bone growth offers the opportunity to isolate a material which could be effective in the stimulation and therapeutic control of bone growth in a wide variety of situations. This is the role now proposed for IGF-II. IGF-II could therefore be useful in disorders of bones caused by bone wasting disorders known as osteopenias, particularly osteoporosis (whether idiopathic or secondary).

By osteopenia is meant disorders of bone such as osteogenesis imperfecta, osteomalacia, osteitis deformans, osteoporosis, rickets, fibrous dysplasia and the like. By osteoporosis is meant idiopathic wasting disorders of bone such as osteoporosis of aging, or osteogenesis imperfects, and secondary osteoporosis (e.g. resulting secondarily to other disease states such as eunuchoidism, hyperthyroidism, Cushing's syndrome, hyperparathyroidism, hypopituita-rism, gluten enteropathy, post-gastrectomy syndrome, glomeruloosteodystrophy, tubular disorders, vitamin D intoxication, immobilization, respiratory acidosis or inadequate dietary intake of vitamin C, calcium, phosphorous or protein). However, it is also envisioned that Insulin-like growth factor-II (IGF-II) could be useful in the treatment of impaired bone growth or in the enhancement of traumatic or surgical fracture repair, wherein surgical fractures include bone implants, or augmentation of bony incorporation of prostheses. Other sources of bone disease include infections (such as tuberculosis and syphilis) and neoplasms (whether bone forming tumours, chondroid tumours or other neoplasms such as Ewing's sarcoma or giant cell tumour).

It is therefore now envisaged that IGF-II could be useful in the treatment of (among other disorders of the bone) osteoporosis.

Osteoporosis is a bone wasting disease characterised by the loss of bone mass leading to atraumatic fracture. There is a resulting rarefaction of bone to leave the skeleton weakened and unable to bear the normal stresses placed upon it. The disease is particularly prevalent in the parts of the skeleton that are weight bearing, especially the spine, wrist and hips. The aetiology of the disease is multi-factorial. One especially common form of the disease occurs after the menopause. In the United States alone, post-menopausal osteoporosis effects 15 million women; it therefore poses a significant health problem.

Because the susceptibility to osteoporosis in a well-defined population, post menopausal women, is particularly high, and because of the osteoblastic enhancing activity of the pharmaceutical agents of the present invention, these agents may be utilized prophylactically to enhance or maintain osteoblastic (bone building) activity in the patient, thereby preventing the onset of the predominant osteoblastic (bone resorbing) activity observed in the disease state. However, forms of osteoporosis exist other than post menopausal, including primary and many forms of secondary osteoporosis. These agents may also be used therapeutically or prophylactically in the treatment of all forms of osteoporosis, whether primary or secondary, and in all bone wasting diseases.

Therefore, according to the invention, there is provided the use of a peptide which is identical to or substantially homologous with an IGF-II, or an active subfragment thereof, in the preparation of an agent for use in human osteoporosis.

The IGF-II is preferably human IGF-II.

Although it may in some circumstances be preferred to use a peptide which is identical to natural human IGF-II, it may not always be essential. The use of homologues or variants is therefore contemplated, as is the use of active subfragments. Post-transcriptional or other modifications (such as glycosylation) are also contemplated. As stated above, the sequence of natural human IGF-II is:

4

AYRPSETLCGGELVDTLQFVCGDRGFYFSRPASRVSRRSRGIVEECCFRS

CDLALLETYCATPAKSE

and this sequence in some embodiments is preferred.

In the treatment of human osteoporosis, IGF-II may be administered parenterally. Therefore, according to a third aspect of the present invention, there is provided a sterile preparation of a peptide which is identical to or substantially homologous with an IGF-II, or an active subfragment thereof.

It has also been discovered that there may be advantages in adminstering IGF-II in combination with a bone localising agent. This may help avoid or at least mitigate any non-specific (extra skeletal) effects of IGF-II.

According to the present invention, therefore, there is provided a composition comprising a peptide which is identical to or substantially homologous with IGF-II, or an active subfragment thereof, and a bone localising agent. A preferred bone localising agent is a fluoride compound. Sodium fluoride is especially preferred. The bone localising agent helps target the IGF-II to bone sites.

According to the present invention there is provided a a pharmaceutical agent for treating an osteopenia in human beings comprising a therapeutically effective amount of Insulin-like Growth Factor II or a therapeutically effective amount of an osteoblastic stimulating fragment of Insulin-like Growth Factor-II, preferably in combination with a potentiating amount of fluoride ion, in a pharmaceutically acceptable carrier.

The pharmaceutical agent is advantageously either a parenteral unit dosage form, or in an aqueous pharmaceutical carrier and suitable for intravenous injection, or in lyophilized form and upon rehydration is suitable for intramuscular administration.

A particularly preferred pharmaceutical agent is one in which the Insulin-like Growth Factor II has the amino acid sequence:

$$Ala-Tyr-Arg-Pro-Ser-Glu-Thr-Leu-Gly-Gly-$$
$$10$$
$$Gly-Glu-Leu-Val-Asp-Thr-Leu-Gln-Phe-Val$$
$$20$$
$$Cys-Gly-Asp-Arg-Gly-Phe-Tyr-Phe-Ser-Arg-$$
$$30$$
$$Pro-Ala-Ser-Arg-Val-Ser-Arg-Arg-Ser-Arg$$
$$40$$
$$Gly-Ile-Val-Glu-Glu-Cys-Cys-Phe-Arg-Ser-$$
$$50$$
$$Cys-Asp-Leu-Ala-Leu-Leu-Glu-Thr-Tyr-Cys$$
$$60$$
$$-Ala-Thr-Pro-Ala-Lys-Ser-Glu$$

or one substantially homologous therewith.

It appears that peptides usable in accordance with the invention may enhance bone cell division, bone matrix production and thereby eventually stimulate bone formation to increase the total bone volume of the body. In combination with a bone localising agent such as fluoride, the peptides may act synergistically to stimulate bone formation.

The present invention is at least to some extent based on the hitherto unpublished discovery that IGF-II is the same as skeletal growth factor (SGF). In particular, human IGF-II has been found to be identical to human SGF. SGF has been implicated in the stimulation of collagen synthesis (Linkhart et al, J. Cell.

Physiol. **128** 307 (1986)) and has been recognised as having a mitogenic effect on bone cells (Farley et al Biochemistry **21** (14) 3509 (1982)).

Although Canalis & Raisz reported some years ago (Cacif. Tissue Int. **29** 33-39 (1979) that Multiplication-Stimulating Factor (MSF) had the effect of stimulating DNA, collagen and non-collagen protein synthesis in bone cultures, this observation on its own is not regarded as significant in view of the large number of substances reported to have a similar effect.

IGF-II may be prepared by a variety of techniques. First, it may be extracted. Extraction may be from plasma as described by Rinderknecht and Humbel (Op. Cit.) Alternatively, it may be isolated from human bones, as described by Mojan et al Biochem. Biophys. Acta **884** 234 (1986)). It should be noted that in the Mojan paper IGF-II was referred to as SGF. IGF-II may also be isolated from bone cells grown in culture.

The second way in which the IGF-II may be prepared is peptide synthesis. As the natural human IGF-II is only 67 amino acids in length, it may be found to be fairly simple to prepare at least small quantities of the peptide by this technique.

The third (and what may ultimately be the preferred) method of preparation, however, involves recombinant DNA techniques. The first stage in such techniques would be to obtain a length of DNA coding for the desired IGF-II. One way to do this would be to isolate mRNA from IGF-II-producing cells and, with the in vitro use of reverse transcriptase, produce cDNA coding for the desired peptide. Alternatively, the DNA may be chemically synthesised. A number of oligonucleotides may be produced, from which the desired cDNA can be prepared by the use of DNA polymerase and DNA ligase. Restriction endonuclease digestion of either end can leave appropriate cohesive restrictions sites for insertion into a plasmid.

Whether the synthetic DNA is cDNA or chemically synthesised, it can either have cohesive ends provided by a restriction endonuclease or it may be terminally tailed by for example oligo-dC by the use of the appropriate nucleotide and terminal transferase.

Whichever tailing method is chosen, a plasmid (for example pBR322) can then be taken and cleaved at a single site by a restriction endonuclease such as PstI. PstI cleaves pBR322 in the gene coding for ampicillin resistance. This allows for easy selection of recombinant plasmids. If desired, the PstI-digested pBR322 can be oligo-dG tailed to complement an oligo-dC tail piece of DNA coding for the desired peptide. The cleaved plasmid and the DNA coding for the peptide can be annealed and ligated; host cells (for example E. coli) can be transformed with the appropriate recombinant plasmid.

The transformed E. coli host cells may be cultured under appropriate conditions to express the IGF-II peptide.

It should be noted that it is not envisaged that production by recombinant DNA techniques should be limited to bacterial systems. Eukaryotic systems such as yeast systems can also be used and may in practice be preferred.

The invention is now illustrated by the following examples.


EXAMPLE 1

Production of IGF-II by bone cells.

Human bone cells are isolated from femoral heads obtained during hip replacement surgery. They are grown in monolayer culture in dishes containing Dulbecco's modified Eagle's medium supplemented with 10% foetal calf serum. The release of IGF-II into the culture medium can be followed using a radioreceptor assay, using rat hepatoma (H35) cells in monolayer culture and IGF-II release is seen to increase with increasing culture time. The results are shown in Table 1.

The production of IGF-II is modulated by systemic agents. In the human bone cell monolayer culture model described above, the addition of insulin (10 to 10,000 ng/ml) for 24 hours stimulates the production of IGF-II by the bone cells in a dose dependent manner. This again can be seen from Table 1.

## TABLE 1

Effect of insulin on the secretion of IGF-II by human bone cells *in vitro*.

| Insulin (ng) | IGF-II levels (ng/ml) after insulin exposure of | |
|---|---|---|
| | 0-24 hours | 24-48 hours |
| 0 | 17.2 + 5.0 | 12.5 + 5.0 |
| 10 | 16.7 + 1.0 | 12.0 + 5.0 |
| 100 | 22.6 + 8.5 | 16.0 + 9.0 |
| 1000 | 24.6 + 6.5 | 19.0 + 11.5 |
| 10000 | 32.0 + 7.0 | 28.0 + 1 |

Treatment of cultured human bone cells with human growth hormone (10 ng/ml) for 5 days increases IGF-II production by 50% ($p < 0.01$). Additionally, human somatostatin (50 ng/ml) also stimulates IGF-II production by human bone cells in culture by 148% compared with untreated controls ($p < 0.01$).

Human bone cells produce very much more IGF-II than IGF-I. Media conditioned by $2 \times 10^4$ cells for 24 hours contain 17.6 ng/ml of IGF-II. During the same interval under identical culture conditions, the cells produced only 1.16 ng/ml of IGF-I. Thus, human bone cells are shown to produce more than 15 times more IGF-II than IGF-I.

EXAMPLE 2

Extraction of IGF-II from bone matrix.

Human femoral heads were obtained during hip replacement surgery at local hospitals. They were cleaned with a knife to remove the adhering soft tissue. The bones were then cut into small pieces (approximately 2 $cm^3$) using a band saw and washed with cold tap water to remove the blood and marrow. The bone pieces were frozen in liquid nitrogen and ground with solid $CO_2$ using a Wiley mill. the resulting bone powder (2 $mm^3$) was washed overnight by constant mixing with deionized water containing proteinase inhibitors (5 mM benzamidine, 100 mM epsilon-aminocaproic acid and 1 mM phenylmethylsulphonyl fluoride). After washing with deionized water, the residue was extracted for 72 to 96 hours by constant stirring with excess (5 vol.) 30 mM tris-acetate/4 M guanidine-HCl (pH 7.4) containing proteinase inhibitors. By this extraction, serum protein (albumin, immunoglobulins etc) and other loosely bound proteins were extracted. The supernatant was decanted and the residue was re-extracted for an additional 24 hours with 4 M guanidine solution. After the guanidine extraction, the residue was demineralised for 7 days by constant mixing with 10% EDTA/4 M guanidine-HCl containing proteinase inhibitors (pH 7.4). The supernatant was decanted, centrifuged (10,000 rpm for 20 minutes), filtered and concentrated by Amicon membrane filtration. The concentrate (guanidine-EDTA extract) was washed with excess of 4 M guanidine-HCl to remove EDTA completely. The guanidine EDTA extract was used for further human IGF-II purification under dissociative conditions. The guanidine EDTA extract was dialysed against distilled water using Spectrapor membrane tubing (molecular weight cut-off 3500), assayed for protein concentration and tested for mitogenic activity.

IGF-II activity in the guanidine-EDTA extract was purified by hydroxyapatite and gel filtration chromatography as follows.

Hydroxyapatite (Fast Flow, Bio-Rad) was equilibrated with 30 mM tris-acetate/4 M guanidine-HCl/10 mM potassium phosphate (pH 7.4). The phosphate concentration of the guanidine-EDTA extract was adjusted to 10 mM and the sample was applied to the hydroxyapatite column (15 x 5 cm). The unbound proteins were eluted with 10 mM phosphate in 30 mM tris-acetate/4 M guanidine-HCl (pH 7.4). The bound proteins were eluted in a single step by increasing the phosphate concentration to 400 mM in the same buffer. Aliquots of unbound and 400 mM phosphate-eluted fractions were desalted by dialysis and tested for protein concentration and mitogenic activity.

The unbound fraction from hydroxyapatite chromatography (which contained 90% of the mitogenic activity) was concentrated and the proteins were separated by HPLC gel-filtration chromatography on a preparative TSK-G3000 SWG column (21.5 x 600 mm, LKB products). The chromatography was performed with a Beckman Model 344 gradient liquid chromatography system, which consists of two Model 112 pumps controlled by a Model 421 controller-programmer. Two ml samples were applied to the column with an Altex Model 210 injection valve and the proteins were eluted at 2 ml/min with 30 mM trisacetate/4 M guanidine-HCl (pH 7.4). The absorbence was monitored at 280 nm (Beckman Model 160 detector) and the elution profile was recorded using a Hewlett Packard Integrator Model 3390A. 2-min fractions were collected and the fractions were pulled according to the protein peaks and concentrated. Aliquots of the pools were dialysed against distilled water to remove guanidine and assayed for protein content and mitogenic activity.

The active pool obtained from gel filtration (6 to 17.5 kDa) was dialysed against 10 mM tris-HCl, pH 7.2 containing 100 mM NaCl and was loaded in 10 ml of the same buffer into a 0.5 x 10 cm heparin-sepharose affinity column (Pharmcia FPLC System). The unbound proteins were eluted with the starting buffer (20 minutes, Flow Rate 1 ml/min, 2 minute fractions) and the bound proteins were eluted by a gradient of 0.1 to 3.0 M sodium chloride over 10 minutes. Fractions were diluted in 1 mg bovine serum albumin per ml DMEM and tested for biological activity using [3H] thymidine incorporation into the DNA of chick embryo bone cells in serum-free monolayer culture as described in Linkhart et al Bone **7** 479-487 (1986).

The active fractions from heparin-sepharose chromatography (fractions 15 to 30, 0.2 to 0.6 M NaCl) were pooled and re-run on the same heparin-sepharose column using the same gradient. The active fractions from the second heparin-sepharose step (fraction 17 to 22) were pooled, concentrated and then subjected to two sequential separations by reverse phase chromatography in 0.1% trifluoroacetic acid (TFA) using a 4.6 x 250 mm C4 column (Bio-Rad RP 304). The first separation used a 10 to 60% acetonitrile gradient in 50 minutes and the second separation used a 25 to 45% acetonitrile gradient in 100 minutes. Final purification of human IGF-II was achieved by HPLC reverse phase chromatography in 0.1% TFA using a 3.9 x 150 mm Microbondapak CN column (Waters Corporation) with a 1-propanol gradient (20 to 40% in 100 minutes). HPLC reverse phase chromatography was done using a Beckman Model 344 gradient liquid chromatography system with a 214 NM detector. Fractions were concentrated by Speed-Vac Evaporation (Savant Instruments).

EXAMPLE 3

IGF-II is active on bone cells.

IGF-II, as obtained in Example 1 or 2, stimulates division in bone cells in monolayer culture and is therefore a bone cell mitogen. This is demonstrated using embryonic chick calvarial cells in monolayer culture. The results are shown in Table 2.

## TABLE 2A

Data are mean $\pm$ standard deviation of six replicates (basal counts = 351 $\pm$ 46, n=6).

| IGF-II (ng/ml) | 3H-TDR INCORPORATION (% of unstimulated control) |
|---|---|
| 0 | 100 $\pm$ 13 |
| 0.3 | 202 $\pm$ 32 |
| 1.0 | 269 $\pm$ 46 |
| 3.0 | 338 $\pm$ 39 |
| 10.0 | 403 $\pm$ 54 |
| 30.0 | 412 $\pm$ 20 |

In a similar experiment conducted on human bone cells, purified human IGF-II at a concentration of 2ng/ml stimulated the proliferation of human bone cells at 362% of the Control value (Table 2B).

**Table 2B.** Effect of Human IGF-II on the Proliferation of Human Bone Cells.

| Treatment | Concentration | CPM | % of Control | Significance |
|---|---|---|---|---|
| Control | | 316 $\pm$ 81 | 100 | |
| human IGF-II[a] | 2 ug/ml | 1146 $\pm$ 80 | 362 $\pm$ 80 | p <.001 |
| serum | 1% | 2479 $\pm$ 816 | 897 $\pm$ 165 | p <.001 |

Data are expressed as mean + 1 SD of six samples, Significance expressed compared to control (Students t Test). a = Partially purified human IGF-II

This enhanced mitogenic activity in the chick and human bone cell cultures, which was stimulated by IGF-II, is directly translatable into enhanced osteoblastic (bone producing) activity within the bone. It is to be noted that when purified human IGF-II was added to cultures of embryonic mouse calvariae pre-labelled with the isotope [45]calcium, IGF-II had no significant effect on the release of the [45]calcium from these bones, indicating a lack of any stimulatory affect on bone resorption.

Thus, by using IGF-II in therapeutically effective amount, it is possible to increase the osteoblastic activity within the bone to a point exceeding the pre-existing osteoclastic activity so as to effect a cure in a patient suffering from an osteopenia.

Similarly, IGF-II can be administered to a patient susceptible to an osteopenia in a prophylactically effective amount, so as to maintain a balance between osteoblastic and osteoclastic activity in said patient.

In a particularly preferred embodiment of the present invention, the osteopenia being treated or prevented is osteoporosis.

EXAMPLE 4

IGF-II when combined with fluoride is targetted to bone.

When labelled with fluoride, IGF-II is bone-specific in its actions. Additionally, when combined with sodium fluoride, IGF-II acts synergistically with this drug to stimulate bone cell division and bone matrix production above the levels achieved by either agent when used alone. Specifically thymidine incorporation into DNA is increased, proline incorporation into collagen is stimulated and cellular alkyline phosphatase is increased by the drug combination.

Thus while both IGF-II and fluoride ion increase the proliferation of human bone cells (Table 2C) as determined by the incorporation of tritiated thymidine into DNA, the combination of IGF-II and fluoride ion produces an unexpectedly enhanced effect upon bone cell proliferation, said fluoride ion potentiating the osteoblastic stimulating activity of IGF-II (Table 2C). In addition, fluoride is the single most effective agent for restoring bone mass lost as a result of osteoporosis, and, at levels that are effective in stimulating bone formation, fluoride particularly as a slow release preparation does not have appreciable side effects on other organ systems.

---

**Table 2C** Effect of IGF-II and Fluoride on Thymidine Incorporation into DNA by Cultured Human Bone Cells and Human Skin Fibroblasts.

| CELL TYPE | PERCENTAGE OF CONTROL | |
|---|---|---|
| | BOVINE IGF-II (8.5 UG/ML) | FLUORIDE (20mM) |
| Human bone | $370 \pm 62$ p .01 | $252 \pm 52$ p <.01 |
| Chick bone | $538 \pm 71$ p .01 | $140 \pm 38$ p <.05 |
| Human skin | $271 \pm 84$ p .01 | $87 \pm 3$ |

Data expressed as mean $\pm$ SD, Control incorporation rates in cpm were $430 \pm 400$ (Human bone), $315 \pm 50$ (chick bone), and $1019 \pm 485$ (human skin).

---

Accordingly, the combination of IGF-II and fluoride ion, wherein the fluoride ion (in a physiologically acceptable form such as MFP, NaF or other) is associated with a pharmaceutically acceptable cation, would provide an especially effective pharmaceutical agent in the treatment of the osteopenias, particularly osteoporosis.

Such pharmaceutically acceptable cations include sodium, potassium, lithium, calcium, magnesium, ferrous, zinc, copper, manganous, aluminum, ferric, manganic and the like. Particularly preferred are the ammonium, potassium, sodium, calcium and magnesium salts.

It is well known in the art that fragments of biologically active polypeptides, either alone or attached to other non-interferring peptides, polysaccharides, aldoses, and like molecules, can possess substantially the same biological activity as the parent intact polypeptide. Accordingly, this invention also encompasses fragments of IGF-II also possessing osteoblastic stimulating activity, whether obtained from an intact molecule or synthetically such as by chemical manipulation or by recombinant DNA techniques.

It is also within the scope of this invention to employ said osteoblastic stimulating fragments alone or in combination with fluoride ion to treat or prevent the osteopenias, particularly osteoporosis, in patients suffering from or susceptible respectively to said diseases states.

A veterinarian or physician of ordinary skill can readily determine whether a subject exhibits a bone wasting condition, osteopenia.

The compounds of this invention may be administered parenterally (other than by mouth) such as intravascularly, intraperitoneally, subcutaneously, intramuscularly, or by suppository using forms known to the pharmaceutical art, or by transdermal route (air gun or skin patch delivery). In the case of fractures, the compounds may, in addition to the above methods, be administered locally, into, at or near the fracture site.

For intravascular, intraperitoneal, subcutaneous, or intermuscular administration, the active drug components of the present invention in liquid, powdered, or lyophilized form may be combined with a suitable diluent or carrier (collectively referred to herein as "carrier" materials) such as water, saline, aqueous dextrose, aqueous buffers, and the like. Preservatives may also be added.

Regardless of the route of administration selected, the compounds of the present invention are formulated into pharmaceutically acceptable dosage forms by conventional methods known to those skilled in the art. The compounds may also be formulated using pharmacologically acceptable acid or base addition salts. Moreover, the compounds or their salts may be used in a suitable hydrated form.

Regardless of the route of administration selected, a non-toxic but therapeutically effective quantity of one or more compounds of this invention is employed in any treatment. The dosage regimen for preventing or treating a bone wasting condition with the compounds of this invention is selected in accordance with a variety of factors, including the type, age, weight, sex, and medical condition of the patient, the severity of the inflammatory condition, the route of administration, and the particular compound employed in the treatment. A physician or veterinarian of ordinary skill can readily determine and prescribe the effective amount of the drug required to prevent or arrest the progress of the condition. In so proceeding, the physician or veterinarian could employ relatively low doses at first and subsequently increase the dose until a maximum response is obtained.

## EXAMPLE 5

Antibody-mediated extraction of IGF-II from bone matrix.

IGF-II antibodies (monoclonal or polyclonal) may be used to extract IGF-II from bone matrix using an affinity column. Alternatively, IGF-I may be used. The reasons that IGF-I antibodies work are because (a) IGF-II is present in a relatively high concentration in bone compared to IGF-I and (b) IGF-II binds to IGF-I antibodies.

## EXAMPLE 6

Purification of IGF-II from serum.

Using conventional techniques, bone cell derived IGF-II may also be purified from animal serum. Mammalian serum, including human, bovine, ovine, porcine or equine serum, may be used.

## EXAMPLE 7

Preparation of Bovine Insulin-like Growth Factor II (bIGF-II) from Extract.

The hip bones from freshly slaughtered cows are mechanically scraped clean of soft tissue and cut into $2cm^3$ sections. The bone sections are frozen in liquid nitrogen and ground in a Wiley mill to yield a bone powder that is washed with warm water to remove fat and serum protein, demineralized and extracted using 20% EDTA, 0.04% sodium azide as described above. The EDTA extracts are concentrated and partially desalted by Amicon ultrafiltration. The remaining EDTA is removed by desalting with Sephadex G-25. Three alternative methods can be used for further purification of the desalted crude extracts.

1. Gel filtration on Agarose yields an active bIGF-II fraction having a MW range of 150 to 200kdal, probably consisting of bIGF-II bound to its binding protein or pre-IGF-II.

2. Direct purification of the crude extract on DEAE Sephacel yields a large bIGF-II fraction that is modestly bound. In addition, active bIGF-II fractions that are unbound or weakly bound to DEAE Sephacel are obtained in the range of 10 to 20kdal.

3. Purification of desalted crude extract on hydroxyapatite yields a non-active fraction that elutes with 0.15M phosphate and an active fractions that is recovered by dissolving the hydroxyapatite support with EDTA. The tightly bound fraction yields approximately equal amounts of large bIGF-II and small bIGF-II.

The large partially purified bIGF-II tends to become small on further manipulation. Evidence suggests that an endogenous bone protease is present in the bone extracts and is activated during the purification process. This protease is evident in the bovine bone extracts as will be indicated subsequently in chicken extracts, but is not observed in the purification of the human extracts.

EXAMPLE 8

Preparation of Chicken Insulin-like Growth Factor (II) (cIGF-II) from Extract.

The tibia and femur of adult chickens are mechanically scraped clean of soft tissue. The cartilage ends are cut off and the bones are frozen in dry ice and smashed into $3mm^3$ pieces. Serum proteins and fat are removed by extensive washing of the bone pieces in 0.03M Tris(acetate), 0.15M NaCl, (pH 7.4), with vigorous agitation. The bone is then subjected to demineralization and extraction with 10% EDTA, 0.04% sodium azide. The EDTA extracts are concentrated and partially desalted by Amicon ultrafiltration and the remaining EDTA removed by Sephadex G-25 chromatography. Two approaches are used to further purify the cIGF-II.

1. Gel filtration of the desalted crude extract on Agarose 0.05M yields predominately a large form of cIGF-II (100 to 200kdal), as well as small quantities of the small cIGF-II in the 10 to 20kdal range.

2. DE-52 chromatography of the desalted crude bone extract yields a cIGF-II fraction that is weakly bound to the matrix. The weakly bound fraction behaves like large cIGF-II when chromatographed on a TSK 3000 HPLC gel filtration column shortly after isolation. Preincubation of the weakly bound fraction before HPLC gel filtration results in a shift of large cIGF-II to the smaller cIGF-II. This change in apparent size is believed to be a result of the presence of a protease which is detected in the fraction weakly bound to DE-52. The cIGF-II activity in the desalted crude extracts binds to hydroxyapatite, QAE cellulose and DE-52, but does not bind to collagen linked to Agarose or to carboxymethyl Sepharose.

EXAMPLE 9

Production of Human Insulin-like Growth Factor - II by Bone Cells in Culture.

Human bone cells were collagenase dissociated and grown in monolayer culture in 24 well dishes containing Dulbecco's modified Eagle's medium (DMEM) supplemented with 10% fetal bovine serum (FBS). HIGF-II is released into the culture medium by the bone cells as they grow.

As noted in the detailed discussion (Table 1), insulin can be added in varying amounts to increase the amount of hIGF-II produced. Similarly, human growth hormone (10 ng/ml) or somatostatin (50 ng/hl) can also be used to enhance hIGF-II production (see detailed discussion).

Extraction and purification are performed as in Example 1.

Two separate in vivo experiments were conducted on rats. In both experiments the rats in the Treated group were parenterally administered a bovine bone IGF-II extract. The rats in the Control group were administered a placebo. The data from Experiment 1 is presented in Tables 3 and 4, that of Experiment 2 in Tables 5 to 7.

Experiment 1

Experiment 1 consisted of 5 rats in the Control group and 3 rats in the Treated group.

In Table 3, the parameters measured in the Treated and Control groups consisted of the following (1) serum calcium; (2) body weight at the start of the experiment; (3) body weight at the end of the experiment; (4) femur alkaline phosphatase activity; and (5) femur acid phosphatase activity. As to the first three parameters, no significant (N.S.) difference was demonstrated between the Control group, which was not receiving IGF-II, and the Treated group, which was receiving bovine bone IGF-II extract.

However, as to parameter 4, the femur alkaline phosphates, there was a significant difference between the Treated and Control groups. Specifically, in the Treated Group, the femur alkaline phosphatase was increased 136% over that in the Control group. Increased alkaline phosphatase activity in bone by itself is suggestive of increased osteoblastic (bone building) activity. However, when combined with the data in Table 4, showing the results of histomorphometric assessment of the rat bones, the result is indicative of increased osteoblastic activity within the bone.

Table 3    In Vivo Effect of Bovine Bone IGF-II Extract on Rats (Experiment 1)

| Parameter | Control | Treated[a] | p[b] |
|---|---|---|---|
| 1. Serum Calcium (mg%) | 9.8(±0.7) | 9.0(±0.5) | NC[c] |
| 2. Body Weight[d] (Start) | 104(±3.7) | 105(±4.2) | NS |
| 3. Body Weight[d] (End) | 147(±0.9) | 147(±9.2) | NS |
| 4. Femur Alkaline Phosphatase (mU/mg protein) | 4.9(±0.9) | 11.6(±1.2) | <0.001 |
| 5. Femur Acid Phosphatase Tartrate Insensitive (mU/mg protein) | 3.4(±0.4) | 4.8(±0.3) | <0.02 |

a    All data reported as mean ±S.E.M. and based on observations from 5 rats in the control group and 3 rats in the treated group.

b    Comparisons of treated versus control were by the students' T test.

c    Not significant at the p <0.05 student T test.

d    in grams

Table 4    Histological Assessment of In Vivo effect of
           Bovine Bone IGF-II Extract on Rats
           (Experiment 1)

| | Parameter | Control | Treated[a] | p[b] |
|---|---|---|---|---|
| 1. | Tibiae Periosteal Bone Formation $(mm^3)$ | 0.23($\pm$0.02) | 0.40($\pm$0.02) | <0.001 |
| 2. | Tibiae Periosteal Bone $(mm^3)$ | 6.75($\pm$0.43) | 8.60($\pm$0.46) | <0.05 |
| 3. | Tibiae Medullary Cavity $(mm^3)$ | 0.67($\pm$0.03) | 0.81($\pm$0.09) | <0.05 |
| 4. | Vertebral Forming Surface[d] (single + double label) % total surface | 58.3($\pm$2.5) | 90.5($\pm$1.7) | <0.001 |
| 5. | Vertebral Neutral Surface[d] (no label) % total surface - V.F.S.) | 41.7($\pm$1.9) | 9.5($\pm$1.4) | <0.001 |

a    All data reported as mean $\pm$S.E.M. and based on
     observations from 5 rats in the control group and
     3 rats in the treated group.

b    Comparisons of treated versus control were by the
     students' T test.

c    Not significant at the p <0.05 student T test.

d    percentage of total surface

The most dramatic effect of IGF-II extracts on bone is seen in the data in Table 4. Table 4 reflects histological comparisons between the tibiae and vertebrae (bones) of the Treated and Control groups of rats from Experiment 1 after sacrifice. The (IGF-II extract) Treated group demonstrates markedly increased bone growth over the Control group in parameters associated with bone growth. Specifically, relative to the Control group, the Treated group demonstrated 73.9% more tibiae periosteal bone formation (the volume of new bone formed in the outer connective tissue, periosteum, covering the bone); and a 27.4% greater periosteal apposition rate (the rate of laying down of new bone) -- parameters 1 and 2 of Table 4.

Moreover, the IGF-II extract Treated group also exhibited a 55% increase in bone forming surface over the untreated Control group, as measured by the ability of the bone forming surface to pick up a label --parameter 4 of Table 4. Alternatively, when this same data is viewed from the perspective of inactive bone forming surface, the data discloses that the Control group had 339% more inactive bone forming surface than the Treated group -- parameter 5 of Table 4.

Thus Experiment 1 demonstrates that parenteral administration of IGF-II extract stimulates significant osteoblastic activity in vivo.

The data in Tables 3 and 4, also suggest that additional activity within the bone may also be stimulated. The increase in tartrate insensitive acid phosphatase activity that was observed in the Treated group is likely due to increased osteoclastic (bone resorbing) activity. However, this was not verified by histological staining. The increase in the tibiae medullary cavities by 21%, parameter 3 of Table 4, is indicative of some increased osteoclastic (bone resorbing) activity.

This increase in osteoclastic activity, however, is due to the presence of small amounts of transforming growth factor (TGF) in the bovine bone extract. Later studies employing pure IGF-II to treat bone cells in culture demonstrate no such osteoclastic effect. Moreover, TGF is recognized as a potent stimulator of osteoclastic (bone resorbing) activity.

Experiment 2

A second more detailed experiment on the in vivo effects of parenteral administration of bovine bone IGF-II extract similarly indicates that IGF-II significantly increases osteoblastic activity within the bone (Tables 5-7).

In this second experiment, Experiment 2, ten rats were used in both the Treated and Control groups. As in Experiment 1, the serum calcium and body weight remained unchanged with treatment (Table 5).

In Experiment 2, the levels of bone alkaline phosphatase were analyzed more extensively. Again, the Treated group exhibited significantly increased levels of alkaline phosphatase in the femur, skull and sternum over that exhibited by the Control group, 83%, 165%, and 102%, respectively -- parameters 1, 2, and 3 of Table 6. The acid phosphatase levels, although increased, were only significantly increased (p <0.001) in the femur and skull of the Treated group and by 93%, and 71% respectively.

The results of the alkaline phosphatase analysis in the femur, skull and sternum are consistent with an increase in osteoblast number and bone formation. The alkaline phosphatase data also agrees very well with the quantitative histology of the tibiae of treated rats, where a 60% increase (p <0.001) in the periosteal bone formation (mm$^3$) and a 40% increase in the matrix apposition rate ( /day) were observed. (Table 7).

As in Experiment 1, the increase in tartrate sensitive acid phosphatase in the femur and skull is consistent with an increase in the amount of osteoblastic acid phosphatase due to the increased number of osteoblasts. It is also consistent with the presence of the osteoclast stimulating agent, beta transforming growth factor (TGF), in the bone extract.

Table 5    In Vivo Effect of Bovine Bone IGF-II Extract
           on Rats (Experiment 2)

| | Parameter | Control[a] | Treated[a] | p[b] |
|---|---|---|---|---|
| 1. | Serum Calcium (mg%) | 10.7($\pm$0.3) | 11.1($\pm$0.3) | NS[c] |
| 2. | Serum Phosphate (mg/dl) | 4.7($\pm$0.5) | 5.8($\pm$0.2) | <0.05 |
| 3. | Serum Alkaline Phosphatase (mU/mg protein) | 63.7($\pm$2.2) | 91.7($\pm$5.2) | <0.001 |
| 4. | Body weight[d] (Start) | 86.4($\pm$0.2) | 85.0($\pm$0.6) | NS |
| 5. | Body Weight[d] | 149.8($\pm$2.6) | 147.7($\pm$2.5) | Ns |

a    All data reported as mean $\pm$S.E.M. and based on
     observations from at least 10 rats in each group.

b    Comparisons of treated versus control were by the
     students' T test.

c    Not significant at p <0.05.

d    in grams

Table 6  Effect of Bovine bone IGF-II Extract on Enzyme Activity within the Bones of Rats. (Experiment 2)

| | Alkaline Phosphatase[a] | | | Acid Phosphatase[a] Tartrate Insensitive | | |
| | (mU/gm bone wet weight) | | | (mU/gm bone wet weight) | | |
| | Control | Treated | p | Control | Treated | p |
|---|---|---|---|---|---|---|
| Femur | 155($\pm$24) | 284($\pm$19) | <0.001 | 31($\pm$5) | 60($\pm$7) | <0.001 |
| Skull | 405($\pm$44) | 1075($\pm$84) | <0.001 | 355($\pm$42) | 607($\pm$52) | <0.001 |
| Sternum | 46($\pm$2) | 93($\pm$7) | <0.001 | 107($\pm$16) | 147($\pm$14) | NS |

a   All data reported as mean $\pm$S.E.M. and based on observations from at least 10 rats in each group.

b   Comparisons of treated versus control were by the students' T test.

c   Not significant at $p < 0.005$.

Table 7    In Vivo Effect of Bovine Bone IGF-II Extract
           on the Tibiae of Rats (Experiment 2)

| Parameter | Control | Treated[a] | p[b] |
|---|---|---|---|
| 1. Tibiae Periosteal Bone Formation $(mm^2)$ | $0.45(\pm0.02)$ | $0.72(\pm0.04)$ | $<0.001$ |
| 2  Tibiae Periosteal Bone Apposition Rate ( /day) | $8.58(\pm0.33)$ | $12.07(\pm0.51)$ | $<0.001$ |

a    All data reported at mean $\pm$S.E.M. and based on observations from at least 10 rats in each group.

b    Comparisons of treated versus control were by the students' T test.

## Claims

1. Use of a peptide which is identical or substantially homologous with IGF II or an active subfragment thereof for the manufacture of a pharmaceutical agent for treating human osteopenia by stimulating bone formation.

2. Use of the peptide according to claim 1 for the manufacture of a pharmaceutical agent for treating human osteoporosis.

3. Use according to claim 1 or 2 wherein the peptide is IGF II.

4. Use according to the claims 1 - 3 wherein the peptide is

Ala-Tyr-Arg-Pro-Ser-Glu-Thr-Leu-Gly-Gly-
10
Gly-Glu-Leu-Val-Asp-Thr-Leu-Gln-Phe-Val
20
Cys-Gly-Asp-Arg-Gly-Phe-Tyr-Phe-Ser-Arg-
30
Pro-Ala-Ser-Arg-Val-Ser-Arg-Arg-Ser-Arg

40
Gly-Ile-Val-Glu-Glu-Cys-Cys-Phe-Arg-Ser-
50
Cys-Asp-Leu-Ala-Leu-Leu-Glu-Thr-Tyr-Cys.
60
-Ala-Thr-Pro-Ala-Lys-Ser-Glu

5. Use of a peptide according to any of claims 1 to 4 in combination with a bone localizing agent.

6. A process for the preparation of a pharmaceutical agent for treating human osteopenia by stimulating bone formation, which can be used according to claim 5, wherein the process comprises mixing a therapeutically effective amount of IGF II, a peptide which is substantially homologous with IGF II or an active subfragment thereof, wherein the process comprises mixing the peptide with a bone localizing agent.

7. Process as claimed in claim 6 wherein the pharmaceutical agent is a parenteral unit dosage form in an aqueous pharmaceutical carrier or suitable for intravenous administration or is in lyophilized form and upon rehydration is suitable for intramuscular administration.

**Patentansprüche**

1. Verwendung eines Peptids welches mit IGF-II identisch oder weitgehend homolog ist oder eines aktiven Unterfragmentes davon zur Herstellung eines Arzneimittels für die Behandlung von Osteopenien in Menschen durch Stimulierung der Knochenbildung.

2. Verwendung des Peptids nach Anspruch 1 zur Herstellung eines Arzneimittels für die Behandlung von Osteoporose beim Menschen.

3. Verwendung nach Anspruch 1 oder 2 dadurch gekennzeichnet, daß das Peptid IGF-II ist.

4.  Verwendung nach einem der Ansprüche 1 - 3 dadurch gekennzeichnet, daß das Peptid


```
        Ala-Tyr-Arg-Pro-Ser-Glu-Thr-Leu-Cys-Gly-
                                               10
        Gly-Glu-Leu-Val-Asp-Thr-Leu-Gln-Phe-Val
                                               20
        Cys-Gly-Asp-Arg-Gly-Phe-Tyr-Phe-Ser-Arg-
                                               30
        Pro-Ala-Ser-Arg-Val-Ser-Arg-Arg-Ser-Arg


                                               40
        Gly-Ile-Val-Glu-Glu-Cys-Cys-Phe-Arg-Ser-
                                               50
        Cys-Asp-Leu-Ala-Leu-Leu-Glu-Thr-Tyr-Cys.
                                               60
        -Ala-Thr-Pro-Ala-Lys-Ser-Glu


        ist.
```


5.  Verwendung eines Peptids nach einem der Ansprüche 1 bis 4 in Kombination mit einem knochenlokalisierenden Mittel.

6.  Verfahren zur Herstellung eines Arzneimittels für die Behandlung von Osteopenia beim Menschen durch die Stimulierung der Knochenbildung, welches nach Anspruch 5 verwendet werden kann dadurch gekennzeichnet, daß das Verfahren das Mischen einer therapeutisch wirksamem Menge von IGF-II, eines Peptids welches weitgehend homolog zu IGF-II ist oder eines aktiven Unterfragmentes davon umfasst, wobei das Verfahren die Mischung des Peptids mit einem knochenlokalisierenden Mittel beinhaltet.

7.  Verfahren nach Anspruch 6 dadurch gekennzeichnet, daß das Arzneimittel eine parenterale Einheitsdosierform in einem wäßrigen pharmazeutischen Träger ist oder in einer Form, die für intravenöse Darreichung geeignet ist oder in lyophilisierter Form und nach Rehydrierung zur intramuskulären Darreichung geeignet ist.

**Revendications**

1.  Utilisation d'un peptide ayant identité ou homologie substantielle avec IGFII ou un subfragment active de celle-ci pour production d'un agent pharmaceutique pour traitement de l'osteopenia humaine en formation d'os.

2.  Utilisation d'un peptide selon la revendication 1 pour production d'un agent pharmaceutique de traitement de l'osteopenia humaine.

3.  Utilisation selon la revendication 1 ou 2 dans lequel le peptide est IGFII.

4.  Utilisation selon les revendications 1 - 3 dans lesquelles le peptide est

```
         Ala-Tyr-Arg-Pro-Ser-Glu-Thr-Leu-Gly-Gly-
                                             10
         Gly-Glu-Leu-Val-Asp-Thr-Leu-Gln-Phe-Val
                                             20
         Cys-Gly-Asp-Arg-Gly-Phe-Tyr-Phe-Ser-Arg-
                                             30
         Pro-Ala-Ser-Arg-Val-Ser-Arg-Arg-Ser-Arg

                                             40
         Gly-Ile-Val-Glu-Glu-Cys-Cys-Phe-Arg-Ser-
                                             50
         Cys-Asp-Leu-Ala-Leu-Leu-Glu-Thr-Tyr-Cys.
                                             60
         -Ala-Thr-Pro-Ala-Lys-Ser-Glu
```

5.  Utilisation d'un peptide selon les revendications 1 - 4 en combination avec un agent de la localisation du os.

6.  Procédé pour la préparation d'un agent pharmaceutique de traitement de l'osteoporosis humaine qui peut utilisé selon le revendication 5, le procédé comprend mixé une quantité de IGFII efficiace therapeutique d'un peptide substantiel homologe avec IGFII ou un subfragment active de celle-ci, lequel procédé comprend mixé le peptide avec un agent de la localisation du os.

7.  Procédé selon la revendication 6 dans lequel l'agent pharmaceutique est un forme unité de dosage parentérale dans un support pharmaceutique aquaeux ou acceptable pour administration intraveineuse ou en forme lyophilisée acceptable pour administration intramusculaire après réhydration.